# EUROPEAN PATENT SPECIFICATION

(11) **EP 2 135 615 B1**
(45) Date of publication and mention of the grant of the patent: **28.01.2015**
(21) Application number: 09152200.3
(22) Date of filing: 06.12.2005
(51) Int. Cl.: A61K 36/00, A61K 31/47, A61K 36/28, A61K 36/758, A61K 36/66

(54) **COMPOSITIONS COMPRISING EXTRACTS FROM SANGUINARIA OR MACLEAYA**
ZUSAMMENSETZUNGEN, ENTHALTEND EXTRAKTE AUS SANGUINARIA ODER MACLEAYA
COMPOSITIONS RENFERMANT DES EXTRAITS DE SANGUINARIA OU MACLEAYA

(30) Priority: 17.12.2004 IT MI20042414
(43) Date of publication of application: 23.12.2009
(62) Divisional of application: 05814992.3
(73) Proprietor: Indena S.p.A., 20139 Milano (IT)
(72) Inventor: Bombardelli, Ezio, 27027, GROPELLO CAIROLI (PV) (IT); Ronchi, Massimo, 20139, MILANO (IT); Di Nicolo', Paola, 20139, MILANO (IT)
(74) Representative: Minoja, Fabrizio

(56) References cited:
- US-A- 4 145 412
- US-A- 5 378 465
- US-A- 5 840 322
- US-B1- 6 264 926
- DATABASE WPI Section Ch, Week 200313 Thomson Scientific, London, GB; Class B04, AN 2003-137579 XP002378506 & KR 2002 073 025 A (LEE Y C) 19 September 2002 (2002-09-19)
- JAYAPRAKASHA G K, TAMIL SELVI, SAKARIAH KK: "Antibacterial and antioxidant activities of grape (Vitis vinifera) seed extracts" FOOD RESEARCH INTERNATIONAL, vol. 36, no. 2, 2003, pages 117-122, XP002378501
- M. SCHLESINGER; EI WEISS; N HOCHMANN, I OFEK, Z ZAKAY-RONES: "Effect of Cranberry Juice Cnstituents on haemagglutination and Infectivity of Influenza Virus" ANTIVIRAL RESEARCH, vol. 57, no. 3, February 2003 (2003-02), page A82, XP002378502
- KNOX Y M; HAYASHI K; SUZUTANI T; OGASAWARA M; YOSHIDA I; SHIINA R; TSUKUI A; TERAHARA N; AZUMA M: "Activity of anthocyanins from fruit extract of Ribes nigrum L. against influenza A and B viruses" ACTA VIROLOGICA, vol. 45, no. 4, 2001, pages 209-215, XP009065720
- CHATURVEDI MADAN M; KUMAR ASHOK; DARNAY BRYANT G; CHAINY GAGAN B N; AGARWAL SUDHA; AGGARWAL BHARAT B: "Sanguinarine (Pseudochelerythrine) is a potent inhibitor of NF-kappaB activation, IkappaBalpha phosphorylation, and degradation" JOURNAL OF BIOLOGICAL CHEMISTRY, vol. 272, no. 48, 28 November 1997 (1997-11-28), pages 30129-30134, XP002378504

## Description

The present invention relates to novel compositions containing anthocyanosides and/or procyanidins in combination with bactericidal isoquinoline alkaloids and optionally with a lipophilic extract of *Zanthoxylum bungeanum* and/or *Echinacea angustifolia.*

### TECHNOLOGICAL BACKGROUND

Throat redness and inflammation with formation of plaques are a frequent aspect of common influenza, cold and other cold diseases. Common cold and influenza, which affect on the average up to three times a year both children and adults, are related to mild viral infections caused by rhinovirus (40%), coronavirus (10%) and to a less extent adenovirus and parainfluenza virus. Although no specific treatments for these pathologies exist, antihistamines and decongestants are considered useful, as the oedema reduction alleviates pain and shortens the duration of the disease underlying inflammation. Said conditions can sometimes involve complications due to secondary bacterial infections, as nasal sinuses are often obstructed by the congestion of mucous membranes wherein pathogenic germs can easily proliferate. In this event, it is necessary to undergo antibiotic treatment.

### DISCLOSURE OF THE INVENTION

The present invention relates to novel compositions containing
a. Procyanidins,
b. Isoquinoline alkaloids extracted from *Sanguinaria canadensis, Macleaya cordata* or *Macleaya microcarpa,* and
c. a lipophilic extract of *Zanthoxylum bungeanum*

According to the present invention, procyanindins preferably derive from *Vitis vinifera* extracts, obtained as described in GB 1541469 or from *Camellia sinensis* extracts, as disclosed in EP 0814823, or other plants containing them, preferably edible plants.

The content in procyanosides- containing extracts can range from 20 to 80 mg per unit dose.

According to the present invention, "isoquinoline alkaloids" mean isoquinoline alkaloids having antibacterial activity, particularly sanguinarines and cheleritrines, preferably obtainable from water-alcoholic extracts of *Sanguinaria canadensis, Macleaya cordata* or *Macleaya microcarpa.* They have broad-spectrum antimicrobial activity. 0.2% Water-alcoholic solutions have shown therapeutic activity in a number of conditions of the upper respiratory tract, acting on pathogenic gram-positive and gram-negative bacteria, mycetes, fungi and protozoa. The extracts containing these alkaloids have shown strong anti-inflammatory topical activity. The extracts containing the isoquinoline alkaloids are typically present in amounts from 2 to 20 mg per unit dose.

The compositions of the invention also comprise lipophilic extracts of *Zanthoxylum bungeanum* enriched in isobutylamides. Said extracts have useful analgesic topical activity through inhibition of nervous conduction. Each one of said extracts can be added to the compositions of the invention in amounts ranging from 0.02 to 0.05 mg per unit dose.

The compositions of the invention will contain, in addition to components a, b, also an isobutylamide-enriched lipophilic extract of *Zanthoxylum bungeanum.* The *Zanthoxylum bungeanum* extract can be prepared e.g. according to WO 00/02570. The compositions of the invention are capable of preventing the formation of purulent plaques infected with various saprophytes of the oral cavity, thus avoiding the use of antibiotics, while reducing the progress of the infection. In particular, the compositions of the invention proved to exert a synergistic effect mainly on the duration of the condition. Furthermore, the compositions of the invention are capable of exerting favourable actions, cleaning the oral cavity and removing dental plaque, thanks to the bacterial adhesiveness-reducing effect, as already mentioned, of both bilberry extracts and procyanindins, and to the high activity of isoquinoline alkaloids on anaerobic bacterial strains.

The pharmaceutical compositions will preferably be formulated as tablets for the slow dissolution in the oral cavity or as chewable forms, particularly gums, which provide the slow release of the active principles. These compositions are used in both prophylactic and curative treatments, as well as for the hygiene of the oral cavity.

Therefore, the present invention also relates to the use of
a. Procyanidins,
b. Isoquinoline alkaloids extracted from *Sanguinaria canadensis, Macleaya cordata* or *Macleaya microcarpa,* and
c. *Zanthoxylum bungeanum* lipophilic extract,
for the preparation of compositions for the prevention and the treatment of bacterial infections of the oral cavity and for the hygiene of the oral cavity.

According to a preferred aspect, the compositions of the present invention will also contain essential oils.

Said compositions will be prepared according to well-known conventional methods, such as those described in "Remington's Pharmaceutical Handbook", Mack Publishing Co., N.Y., USA, together with suitable excipients.

The following example illustrates the invention in detail.

### EXAMPLE I - 1000 mg chewable tablets containing

| | | |
|---|---|---|
| 1. | *Vitis vinifera* alcoholic extract | 80 mg |
| 2. | *Sanguinaria canadensis* alcoholic extract | 2 mg |
| 3. | *Zanthoxylum bungeanum* extracted purified | 0.025 mg |
| 4. | Soy lecithin | 30 mg |
| 5. | Anhydrous citric acid | 5 mg |
| 6. | L-Cysteine | 5 mg |
| 7. | Lactose | 200 mg |
| 8. | Mannitol | 512.475 mg |
| 9. | Methylcellulose | 40 mg |
| 10. | Glycerol palmitostearate | 50 mg |
| 11. | Berry flavours | 40 mg |
| 12. | Potassium acesulfame | 0.5 mg |
| 13. | Talc | 10 mg |
| 14. | Sodium bicarbonate | 25 mg |

## Claims

1. Compositions containing a combination of:
a. procyanidins extracted from *Vitis vinifera,*
b. Sanguinarines and cheleritrines extracted from *Sanguinaria canadensis, Macleaya cordata* or *Macleaya microcarpa,*
and
c. *Zanthoxylum bungeanum* lipophilic extract.

2. Compositions as claimed in claim 1 in the form of tablets or chewing gums.

3. The use of a combination of:
a. procyanidins extracted from *Vitis vinifera,*
b. Sanguinarines and cheleritrines extracted from *Sanguinaria canadensis, Macleaya cordata* or *Macleaya microcarpa,* and
c. *Zanthoxylum bungeanum* lipophilic extract,
for the preparation of compositions for the prevention and the treatment of bacterial infections of the oral cavity.

4. The use of a combination of:
a. procyanidins extracted from *Vitis vinifera,*
b. sanguinarines and cheleritrines extracted from *Sanguinaria canadensis, Macleaya cordata* or *Macleaya microcarpa,* and
c. *Zanthoxylum bungeanum* lipophilic extract,
for the preparation of compositions for the hygiene of the oral cavity.

## Patentansprüche

1. Zusammensetzungen enthaltend eine Kombination von:
a. Procyanidinen, extrahiert aus *Vitis vinifera,*
b. Sanguinarinen und Cheleritrinen, extrahiert aus *Sanguinaria canadensis, Macleaya cordata* oder *Macleaya microcarpa,*
und
c. lipophilem Extrakt von *Zanthoxylum bungeanum*.

2. Zusammensetzungen nach Anspruch 1 in Form von Tabletten oder Kaugummis.

3. Verwendung einer Kombination von:
a. Procyanidinen, extrahiert aus *Vitis vinifera,*
b. Sanguinarinen und Cheleritrinen, extrahiert aus *Sanguinaria canadensis, Macleaya cordata* oder *Macleaya microcarpa,*
und
c. lipophilem Extrakt von *Zanthoxylum bungeanum,* zur Herstellung von Zusammensetzungen zur Vorbeugung und Behandlung bakterieller Infektionen der Mundhöhle.

4. Verwendung einer Kombination von:
a. Procyanidinen, extrahiert aus *Vitis vinifera,*
b. Sanguinarinen und Cheleritrinen, extrahiert aus *Sanguinaria canadensis, Macleaya cordata* oder *Macleaya microcarpa,*
und
c. lipophilem Extrakt von *Zanthoxylum bungeanum,*
zur Herstellung von Zusammensetzungen für die Hygiene der Mundhöhle.

## Revendications

1. Compositions contenant une combinaison :
a. de procyanidines extraites de *Vitis vinifera,*
b. de sanguinarines et des chéléritrines extraites de *Sanguinaria canadensis, Macleaya cordata* ou *Macleaya microcarpa,* et
c. d'un extrait lipophile de *Zanthoxylum bungeanum.*

2. Compositions selon la revendication 1, sous la forme de comprimés ou de chewing-gums.

3. Utilisation d'une combinaison :
a. de procyanidines extraites de *Vitis vinifera,*
b. de sanguinarines et de chéléritrines extraites de *Sanguinaria canadensis, Macleaya cordata* ou *Macleaya microcarpa,* et
c. d'un extrait lipophile de *Zanthoxylum bungeanum,*
pour la préparation de compositions destinées à la prévention et au traitement d'infections bactériennes de la cavité buccale.

4. Utilisation d'une combinaison :
a. de procyanidines extraites de *Vitis vinifera,*
b. de sanguinarines et de chéléritrines extraites de *Sanguinaria canadensis, Macleaya cordata* ou *Macleaya microcarpa,* et
c. d'un extrait lipophile de *Zanthoxylum bungeanum,*
pour la préparation de compositions destinées à l'hygiène de la cavité buccale.
